Europäisches Patentamt

European Patent Office

Office européen des brevets

(11) Veröffentlichungsnummer: **0 287 549 B1**

# EUROPÄISCHE PATENTSCHRIFT

(45) Veröffentlichungstag der Patentschrift:
**18.12.91 Patentblatt 91/51**

(51) Int. Cl.⁵ : **H05B 6/80**

(21) Anmeldenummer: **88890047.9**

(22) Anmeldetag : **09.03.88**

(54) **Vorrichtung zum Erhitzen von Gegenständen und Organismen.**

(30) Priorität : **14.04.87 AT 932/87**

(43) Veröffentlichungstag der Anmeldung :
**19.10.88 Patentblatt 88/42**

(45) Bekanntmachung des Hinweises auf die
Patenterteilung :
**18.12.91 Patentblatt 91/51**

(84) Benannte Vertragsstaaten :
**AT BE CH DE ES FR GB GR IT LI LU NL SE**

(56) Entgegenhaltungen :
**EP-A- 0 069 105**
**EP-A- 0 116 921**
**EP-A- 0 198 430**
**WO-A-86/04206**
**DE-A- 1 947 712**
**DE-A- 3 430 673**
**DE-B- 1 183 615**
**FR-A- 1 471 131**
**FR-A- 2 250 254**
**US-A- 3 402 277**
**US-A- 3 436 508**
**US-A- 3 651 300**
**US-A- 4 417 116**

(73) Patentinhaber : **Katschnig, Helmut, Dr.**
**Burggasse 108**
**A-8750 Judenburg Steiermark (AT)**

(72) Erfinder : **Katschnig, Helmut, Dr.**
**Burggasse 108**
**A-8750 Judenburg Steiermark (AT)**
Erfinder : **Mooshammer, Wolfgang, Dipl.-Ing.**
**Burggasse 108**
**A-8750 Judenburg Steiermark (AT)**
Erfinder : **Bischoff, Christian**
**Burggasse 108**
**A-8750 Judenburg Steiermark (AT)**
Erfinder : **Berger, Erwin**
**Burggasse 108**
**A-8750 Judenburg Steiermark (AT)**

(74) Vertreter : **Casati, Wilhelm, Dipl.-Ing. et al**
**Patentanwälte Casati, Wilhelm, Dipl.-Ing. Itze,**
**Peter, Dipl.-Ing. Amerlingstrasse 8**
**A-1061 Wien (AT)**

**Beschreibung**

Die Erfindung beschäftigt sich mit Vorrichtungen zum gleichmäßigen Erwärmen von Gegenständen bzw. Inaktivieren von eiweiß- bzw. nukleinsäurehältigen Organismen, durch Einwirken von mittels Magnetrons erzeugten Mikrowellen in einem Raum, in den die Gegenstände bzw. die die Organismen aufweisenden Gegenstände einbringbar sind.

Durch die DE-A1-3430673 wurde ein Durchlaufverfahren zum Sterilisieren bekannt, bei dem das zu sterilisierende Gut durch einen Hohlleiter, zwischen zwei synchron laufenden Förderbändern liegend, hindurchbewegt wird. Derartige Hohlleiter besitzen kalte Stellen, so daß die Forderung nach einem vollkommenen Sterilisieren mit der bekannten Einrichtung praktisch nicht erfüllbar ist. Abgesehen davon ist die Vorrichtung kompliziert aufgebaut und bringt Probleme bei der Abschirmung nach außen.

In der US-Patentschrift US-A-3402277 wird ein Mikrowellenbehandlungstunnel beschrieben, in welchem pulverförmiges oder granuliertes Material oder auch Stückgüter behandelt werden können, u.zw. dadurch, daß sie mittels eines Fördergurtes durch die Heizzone geführt werden. Das zu behandelnde Gut wird dabei jeweils nur von Wellen eines einzigen Mikrowellengenerators behandelt, wobei jedoch dadurch, daß das Gut mittels des Fördergurtes an mehreren Mikrowellengeneratoren vorbeigeführt wird, zu verschiedenen Zeiten an unterschiedlichen Stellen das Gut behandelt wird, nämlich aufeinanderfolgend von links, von rechts, von oben und von unten. Damit wird das Gut nicht gleichzeitig allen erzeugten Mikrowellen ausgesetzt, sondern jeweils aufeinanderfolgend, d.h. zeitlich gegeneinander versetzt, an verschiedenen Stellen seiner Oberfläche. Damit können aber kalte Stellen bei der Behandlung des Gutes in der bekannten Vorrichtung nicht vermieden werden. Die bekannte Anordnung schließt darüberhinaus, weil es sich um einen Tunnel handelt, also um ein Element, das an seinen beiden Enden offen ist, jede Kontrolle von Temperatur und Feuchtigkeit aus. Diese Parameter sind an den Tunnelenden sicher verschieden von den Werten im Zentrum des Tunnels. Für ein wirkungsvolles Erwärmen der Gegenstände auf eine gleichmäßige Temperatur und damit beim Erwärmen von Gegenständen, die eiweiß- bzw. nukleinsäurehältige Organismen enthalten, ein sicheres Abtöten bzw. Inaktivieren dieser Organismen, ist die bekannte Vorrichtung dabei weder vorgesehen, noch ist sie dafür geeignet. Der Tunnel der bekannten Vorrichtung besitzt an zwei einander zugekehrten Flächen jeweils zwei Magnetrons, die jedoch in Längsrichtung des Tunnels gegeneinander versetzt sind. Der Querschnitt des Tunnels senkrecht zu seiner Längserstreckung ist als unregelmäßiges Oktogon ausgebildet. Die Magnetrons sind bei der bekannten Vorrichtung außerhalb des Tunnels angeordnet. Der Außenraum steht mit dem Tunnelinneren nicht in Verbindung. Die Magnetrons der bekannten Vorrichtung unterliegen keiner Zwangskühlung.

Die DE-A-1947712 offenbart einen in Form eines sogenannten Kuppeldomes ausgebildeten Resonanzraum, der an einer Seite dadurch elektrisch offengehalten ist, daß die dort befindliche Wand aus für Mikrowellen durchlässigem Material, z.B. aus einer Teflonfolie, besteht. Unter Verwendung dieses Kuppeldomes sollen Mikrowellen derart erzeugt werden, daß die zu behandelnden Gegenstände gleichmäßig erwärmt werden können. Wo und in welcher Weise diese Erwärmung konkret erfolgen soll, ist jedoch dem Dokument nicht zu entnehmen. Insbesondere fehlen Ausführungen über den Behandlungsraum, so daß dem Dokument nichts zu entnehmen ist, was auf eine Eignung des geoffenbarten Gerätes zur gleichmäßigen Erhitzung von Gegenständen, wie dies vor allem auch für die Tötung von Keimen und Viren unerläßlich ist, schließen läßt.

Durch die DE-B-1183615 wurde eine Mikrowellenheizvorrichtung bekannt, bei der ein Hohlraumresonator vorgesehen ist, in welchen die zu erhitzenden Gegenstände eingebracht werden können, wobei in diesen Hohlraumresonator zwei Magnetrons hineinragen. Diese Magnetrons sind entweder in einer Deckfläche des Hohlraumresonators angeordnet, u.zw. nebeneinander liegend. In einer anderen Ausführungsform ist ein Magnetron an der Deckfläche und ein weiteres Magnetron an der dieser Deckfläche benachbarten, unter 90° zur Deckfläche stehenden Seitenfläche angeordnet. Es können auch mehr als zwei Magnetrons als Energiequellen verwendet werden, wobei jedoch Angaben über deren Anordnung fehlen. Anwendungsgebiet der bekannten Anordnung dürften u.a. Backöfen sein. Mit der aus dem Dokument bekannten Anordnung soll zwar eine verhältnismäßig gleichförmige Energiedichte im ganzen Ofen erreicht werden, wozu der Ofen vorzugsweise abwechselnd in zwei verschiedenen Schwingungsformen angeregt wird. Die Maßnahmen, mit welchen dieses Ziel erreicht werden soll, d.h. die Anordnung der Magnetrons, sind für zwei Magnetrons wie vorstehend erwähnt geoffenbart. Eine solche Anordnung vermeidet das Entstehen von kalten Stellen nicht.

Aus der Publikation EP-A2-0116921 wurde es bekannt, eine Injektionsnadel, über welche Behandlungsflüssigkeit aus einem verschlossenen Behältnis einem Patienten zugeführt werden soll, in der Weise zu sterilisieren, daß nach dem Perforieren des behälterverschlusses, Flüssigkeit durch die Nadel aus dem Behälter in den bereich der Nadel gebracht und durch Mikrowellenenergie erhitzt wird, um die Nadel zu sterilisieren. Die bekannte Vorrichtung ist auf den vorstehend dargelegten Verwendungszweck beschränkt.

In der FR-Patentschrift FR-A-2250254 ist ein quaderförmiger Schmelzofen beschrieben, dem durch Mikrogeneratoren Energie zugeführt wird. Der quaderförmiger Raum, der den Mikrowellen ausgesetzt wird, ist durch sechs Wandungen begrenzt, von welchen die obere eine zentrale Öffnung besitzt, durch welche mit Spiel ein Rezipient hindurchragt, der das zu schmelzende Gut aufnimmt. Anschließend an die vorerwähnte durchbrochene Wandung kann dann das im Rezipienten befindliche Gut der Einwirkung von Mikrowellenenergie ausgesetzt werden. Der Rezipient ist durch ein Sieb in einen oberen und einen unteren Abschnitt geteilt, wobei im unteren Abschnitt eine vom oberen Abschnitt unterschiedliche Energiedichte herrscht, da im unteren Abschnitt lediglich der Schmelzzustand aufrecht zu erhalten ist. Bei dem in dem französischen Dokument beschriebenen Schmelzofen kommt es auf die Vermeidung von kalten Stellen nicht an. Es ist lediglich sicherzustellen, daß das Gut, das sich oberhalb des Siebes befindet, geschmolzen wird. Ob dabei einige Stellen kalt bleiben und das ungeschmolzene Gut im Zuge des Absinkens in dem Raum oberhalb des Siebes zu einem späteren Zeitpunkt geschmolzen wird als ein anderes Partikel, ist für die Funktion vollkommen bedeutungslos.

Für medizinischen Abfall als Träger der Organismen (Bakterien, Viren, Sporen) weist keines der Dokumente einen Weg zur Desinfizierung, wiewohl es bekannt ist, daß infizierter Abfall vor seiner endgültigen Entsorgung (z.B. auf einer Deponie oder in einer Müllverbrennung) desinfiziert werden muß, um eine Gefährdung des Entsorgungspersonals bzw. der Allgemeinheit zu unterbinden. Es ist bekannt, daß sich lebende Zellen und Mikroorganismen, ebenso wie die noch kleineren Viren und sonstigen eiweß- und nukleinsäurehältigen Organismen, durch Einwirkung von chemischen und physialkischen Noxen abtöten bzw. inaktivieren lassen, wodurch sie ihre Pathogenität verlieren. Die Entkeimung von infiziertem Material kann durch physikalische Methoden, wie thermische Inaktivierung, Inaktivierung durch Strahleneinwirkung (β-, Röntgen-, γ-, UV-Strahlen) und durch Spezialfiltration, bei der die Keime zurückgehalten werden, erfolgen. Bei der thermischen Inaktivierung werden unter dem Einfluß von Hitze Eiweißkörper bzw. Nukleinsäuresequenzen, welche Lebewesen im weitesten Sinne enthalten, irreversibel geschädigt, wodurch alle Wachstums- und Vermehrungsfunktionen des jeweils betroffenen Organismus sicher ausgeschaltet werden. Thermische Inaktivierungsmethoden stehen heute weltweit im medizinischen und technischen bereich wegen ihrer sicheren Wirkung, ihrer genauen Standardisierbarkeit und ihrer guten Praktikabilität im Routineeinsatz und werden auch bei nicht näher spezifiziertem infektiösem Abfall häufig verwendet, um krankheitserregende Keime (Bakterien, Viren, Sporen) vor der endgültigen Entsorgung (Deponie oder Verbrennung) zu vernichten und eine Gefährdung des Entsorgungspersonals sowie der Allgemeinheit zu unterbinden.

Die verwendeten Geräte arbeiten entweder nach dem Verfahren der Heißluftsterilisation oder der Autoklavierung. Bei beiden Verfahren muß Hitze bzw. der gespannte Dampf von außen an das Sterilisationsgut herangeführt werden, weshalb nur unter Einhaltung exakter Beschickungsvorschriften auch zielführende Sterilisationsergebnisse zu erreichen sind.

Der Vorteil dieser beiden Methoden liegt in der Sicherheit und Vollständigkeit der Desinfektion (Sterilisation), der Nachteil in einem enorm hohen Aufwand an Energie und Zeit, sowie in einer unvermeidlichen, unangenehmen Geruchsbelastung der näheren Umgebung.

Die Inaktivierung durch Strahlen, insbesondere durch radioaktive Strahlen, ist für die Desinfektion (Sterilisation) infizierten Abfalls wegen des hohen technischen und sicherheitstechnischen Aufwandes kaum praktikabel.

Ähnliches gilt auch für die Spezialfiltration, bei der die Keime im Filter in besonders hoher Konzentraton anfallen und dann beseitigt werden müssen. Abgesehen davon ist diese Methode hauptsächlich auf Flüssigkeiten, gegebenenfalls auch Gase, beschränkt, kann jedoch für Keime keine Anwendung finden, wenn sich die Keime auf festen Trägern befinden.

Neben den vorerwähnten physikalischen Methoden können auch chemische Methoden zur Entkeimung eingesetzt werden. Chemische Methoden, bei welchen desinfizierende Chemikalien eingesetzt werden, eignen sich vor allem zur Oberflächendesinfektion und zur Desinfektion von Innenwänden von Hohlräumen, in die gezielt Desinfektionsmittel eingebracht werden können.

Wie vorstehend erwähnt, wurde es auch bekannt, Mikrowellenenergie einzusetzen, um Keime abzutöten. Dabei erfahren alle der Mikrowellenenergie ausgesetzten lebenden Strukturen eine Erhitzung des Körperwassers von innen her bis über den Siedepunkt ("kontaktlose" Wärmezufuhr), wodurch eine Denaturierung — Zelltod — erreicht wird. Wesentlich für die Mikrowellensterilisation ist der Umstand, daß im Sterilisationsraum alle dort vorhandenen Keime der Mikrowellenenergie ausgesetzt werden. Bei herkömmlichen Geräten sind keine Vorkehrungen zur Vermeidung sogenannter "kalter" Stellen getroffen. Die Vermeidung solcher kalter Stellen ist auch wesentlich, wenn im Resonanzraum Gegensände eingebracht sind, die gleichmäßig erwärmt werden sollen. Da die Mikrowellenenergie immer nur kurzzeitig einwirkt, ist eine Erhitzung durch Wärmeleitung im Gegenstand selbst praktisch bedeutungslos.

Aufgabe der Erfindung ist es nun, eine Vorrichtung zu schaffen, bei deren Anwendung das Auftreten kalter Stellen vermieden wird. Dies ist für eine gleichmäßige Erwärmung von Gegenständen, vor allem, wenn solche Gegenstände abzutötende Organismen enthalten, von Bedeutung. Diese Aufgabe wird in einer erfindungsgemäßen Vorrichtung zum Erwärmen von Gegenständen durch Einwirken von mittels mehrerer Magnetrons erzeugten Mikrowellen gelöst, die über Wellenleiter in einen Raum eingespeist werden, in den die zu erwärmenden Gegenstände einbringbar sind, wobei die Wellenleiter an einen Hohlresonator angeschlossen sind und der von einem rechteckigen Prisma gebildete Raum zur Aufnahme der zu erwärmenden Gegenstände vom Hohlresonator umschlossen ist, wobei eines der Magnetrons jeweils in den von den Wellen des bzw. der anderen Magnetrons freien Raumbereich strahlt und die Vektoren der elektrischen Feldstärke der in den Hohlresonator eingekoppelten Wellen orthogonal bzw. annähernd orthogonal zueinander angeordnet sind und die Richtung eines jeden der Vektoren der eingekoppelten Wellen von der Richtung einer der Achsen des durch drei senkrecht aufeinanderstehende Kanten des den Raum zur Aufnahme der zu erwärmenden Gegenstände bildenden rechteckigen Prismas bestimmten räumlichen Koordinatensystems abweicht, so daß im Hohlresonator kalte Stellen vermieden werden und das in den Hohlresonator eingebrachte Gut zur Gänze der Einwirkung der Mikrowellen ausgesetzt wird. Bei Verwendung der wie vorstehend beschrieben, erfindungsgemäß gestalteten Vorrichtung kann somit sichergestellt werden, daß sich im Hohlresonator keine kalten Stellen ausbilden. Das Gerät ist damit auch für die Sterilisation medizinischer Geräte, für die Entsorgung medizinischen (infektiösen) Abfalls, für die Sterilisation von OP-Wäsche oder Verbandsstoffen geeignet, jedoch können auch Flüssigkeiten und auch Gase, die z.B. in Schläuchen kontinuierlich durch den Hohlresonator geleitet werden, sterilisiert werden, wie überhaupt eine gleichmäßige Erwärmung von in den Raum eingebrachten Gegenständen sichergestellt ist.

In weiterer Ausgestaltung der Erfindung wird vorgeschlagen, daß drei Magnetrons vorgesehen sind, von welchen zwei an einer Seitenwand des Hohlresonators liegen und das dritte Magnetron an der dieser Wand gegenüberliegenden Wand des Hohlresonators liegt. Dies stellt eine besonders günstige Anordnung der Magnetrons zur Vermeidung kalter Stellen dar.

Um die betriebssicherheit weiter zu erhöhen, ist es zweckmäßig, wenn der Innenraum des Hohlresonators über, insbesondere im bereich des Bodens des Resonators befindliche, Öffnungen mit Außenräumen verbunden ist, in welchen insbesondere Raumluft ansaugende Ventilatoren zur Belüftung der Außenräume und der dort angeordneten Magnetrons samt zugehörigen Hohlleitern, über welche die Wellen in den Hohlresonator eingekoppelt werden, angeordnet sind. Die Ventilatoren sind damit sehr leicht einer Inspektion zugänglich und werden zur Luftumwälzung im Innenraum des Hohlresonators benutzt.

Eine weitere Ausgestaltung der erfindungsgemäßen Vorrichtung sieht vor, daß der Innenraum des Hohlresonators über einen, bevorzugt in das Kanalnetz einmündenden, Auslaßkanal mit einem Abluftkanal verbindbar ist, wobei im Auslaßkanal eine Stauklappe angeordnet ist. Dadurch können alle bei einer Desinfektion (Sterilisation), jedoch auch bei einer Erhitzung anderer Gegenstände entstehenden Dämpfe und Gerüche abgeleitet werden, bevorzugt in das Kanalisationssystem, so daß keine Geruchsbelästigung für das Bedienungspersonal bzw. für die Umgebung entsteht.

In der erfindungsgemäßen Vorrichtung kann weiters mit dem Innenraum des Hohlresonators ein Meßkanal verbunden sein, der aus dem Innenraum des Hohlresonators, insbesondere über einen Saugventilator, mit Luft versorgbar ist und die Luft über eine Heizeinrichtung einem Feuchtesensor zuführt.

Die Heizeinrichtung und der Feuchtesensor können in dem Meßkanal angeordnet sein. Diese Ausgestaltung der Vorrichtung erlaubt es, die Luftfeuchtigkeit im Resonanzraum kontinierlich zu messen, wodurch vor allem die für das Sterilisieren wichtige Überwachung der Desinfizierbarkeit (Sterilisierbarkeit) und der Desinfektion (Sterilisation) sowie die Steuerung des Funktionsablaufes ermöglicht wird. Nicht mittels Mikrowellen erwärmbares, z.B. trockenes, Gut kann als solches erkannt werden. Sobald nämlich der Feuchtegehalt auf einen, sich vom Anfangsfeuchtegehalt vor der Magnetroneinschaltung signifikant unterscheidenden Wert steigt, läßt sich darauf schließen, daß das Gut mindestens die Kochtemperatur von Wasser erreicht hat. Somit kann eine Aussage darüber getroffen werden, ob sich überhaupt durch Mikrowellen erwärmbares Gut im Behältnis befindet. Sind Heizeinrichtung und Feuchtesensor im Meßkanal angeordnet, ergibt sich eine kompakte Bauform und die Möglichkeit, die Feuchte nahe dem Ort des Entstehens, nämlich möglichst nahe dem Innenraum, festzustellen.

In weiterer Ausgestaltung der erfindungsgemäßen Vorrichtung mündet der Meßkanal in den Abluftkanal. Damit wird sichergestellt, daß selbst die geringe, für die Bestimmung des jeweiligen Feuchtegehaltes erforderliche Luftmenge aus dem Innenraum nicht in die die Sterilisationseinrichtung umgebende Luft gelangt.

Der Feuchtesensor kann bei einer besonders zweckmäßig gestalteten erfindungsgemäßen Vorrichtung mit einer Abschalteinrichtung für die Energieversorgung der Magnetrons gekoppelt sein. Dadurch ist es möglich, die Vorrichtung abzuschalten, wenn das darin befindliche Gut durch Mikrowellenenergie nicht erwärmbar und damit auch nicht sterilisierbar ist. Der Feuchtesensor müßte bei Vorhandensein von durch Mikrowellen erhitzbarem und damit auch von sterilisierfähigem Gut im Hohlresonator einen Anstieg der Feuchte nach Inbetriebnahme der Einrichtung melden. Wird ein solcher Anstieg nicht gemeldet, erfolgt die Abschaltung. Am Gerät kann dann bevorzugt selbsttätig kenntlich gemacht werden, daß sich nicht desinfizierbares (sterilisierbares) Gut im Resonanzraum befindet.

Für die Betriebssicherheit der erfindungsgemäßen Vorrichtung ist es zweckmäßig, wenn ein mit dem Rauminneren des Hohlraumes verbundenes Thermorelais zum Schutz gegen Überhitzung vorgesehen ist. Damit kann sichergestellt werden, daß weder die Vorrichtung als solche, noch die eingebrachten Gegenstände Hitzeschäden erleiden und die Hitzewirkung auch zum Abtöten der Keime wirksam werden kann.

Soferne im Hohlresonator auch Dampf erzeugt wird, ist es zweckmäßig, am Hohlresonator ein Sicherheitsventil zur Sicherung des Hohlresonators gegen inneren Überdruck vorzusehen. Eine Zerstörung des Gerätes und damit ein Austritt von Mikrowellenenergie in den umliegenden Raum und auch ein Austritt von Keimen kann hiebei sicher ausgeschlossen werden.

In Weiterbildung der Erfindung kann im Innenraum des Hohlresonators eine autarke, somit von einem Außenanschluß unabhängige Flüssigkeitsquelle, z.B. in Form eines mit Wasser, gegebenenfalls zusätzlich mit Desinfektionsmittel und/oder Duftstoffen befüllten Beutels angeordnet werden. Der Beutel bzw. das Behältnis für die Flüssigkeit darf nach außen nicht dampfdicht verschlossen sein.

Die Erfindung wird nachstehend anhand der Zeichnung näher erläutert. Es zeigen,

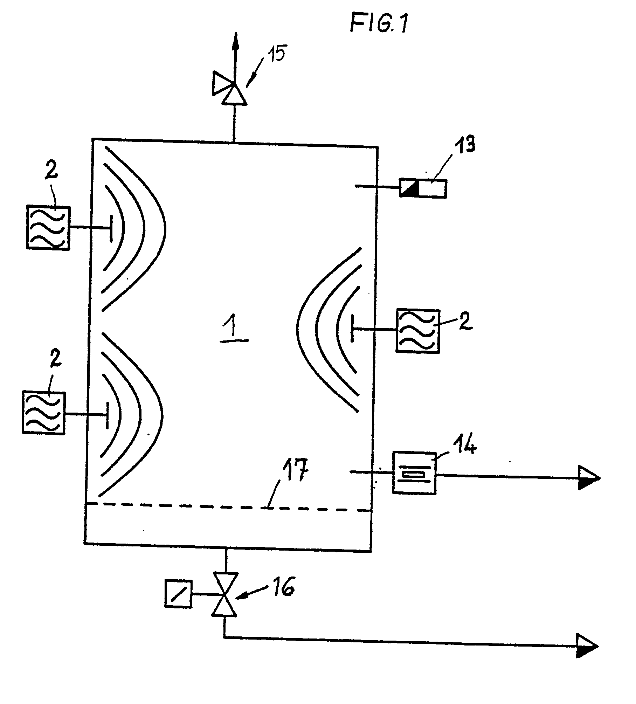

Fig. 1 schematisch in einer Vorderansicht ein erstes Ausführungsbeispiel einer erfindungsgemäßen Vorrichtung,

Fig. 2 ebenfalls in einer schematischen Vorderansicht eine zweite Ausführungsform,

Fig. 3 einen Schnitt entlang der Linie III-III in Fig. 2.

In der Zeichnung ist mit 1 ein Hohlresonator bezeichnet, der mittels mehrerer Magnetrons 2 mit Mikrowellenenergie versorgt wird. Eines der Magnetrons 2 strahlt dabei jeweils in den von den Wellen des bzw. der anderen Magnetrons 2 freien Raum. Die energetischen Wellen der Magnetrons 2 bestreichen in der vom Hohlresonator 1 umschlossenen Kammer unter Vermeidung von Energieschatten alle Raumbereiche gleichmößig. Kalte Stellen werden solcherart im Hohlresonator 1 vermieden, so daß in den Hohlresonator durch eine durch eine Tür od.dgl. Verschlußorgan verschließbare Öffnung eingebrachtes Gut zur Gänze der Einwirkung der Mikrowellen ausgesetzt wird. Zur Verbesserung der Wirksamkeit der Mikrowellen kann in die beschichte Kammer des Resonators 1 kalter Wasserdampf (erzeugt über einen außen liegenden Ultraschallvernebler 14) eingeleitet werden, der dann durch Einwirkung der Mikrowelle erhitzt, zusätzlich seine Wirkung von außen her auf das zu behandelnde (zu sterilisierende) Material entfalten kann. An Sicherheitseinrichtungen sind ein Sicherheitsventil 15, das sich bei Dampfdrücken, die über tolerablen (und erwünschten) Grenzen liegen, öffnet und ein Thermorelais 13 vorgesehen, das als Überhitzungsschutz dient. Am Boden der Kammer befindet sich ein regelbares Ablaßventil 16. Der gesamte Programmablauf des Sterilisationsvorganges kann über eine (nicht dargestellte) elektronische Steuereinheit geregelt werden. Oberhalb des Bodens der Kammer des Hohlresonators 1 befindet sich ein Rost 17, auf dem ein Behälter, der das zu sterilisierende Gut enthält, abgestellt werden kann. Wie den Zeichnungen zu entnehmen ist, sind drei Magnetrons 2 vorgesehen, von welchen zwei an einer Seitenwand des Hohlresonators 1 liegen und das dritte Magnetron 2 an der dieser Wand gegenüberliegenden Wand des Hohlresonators liegt.

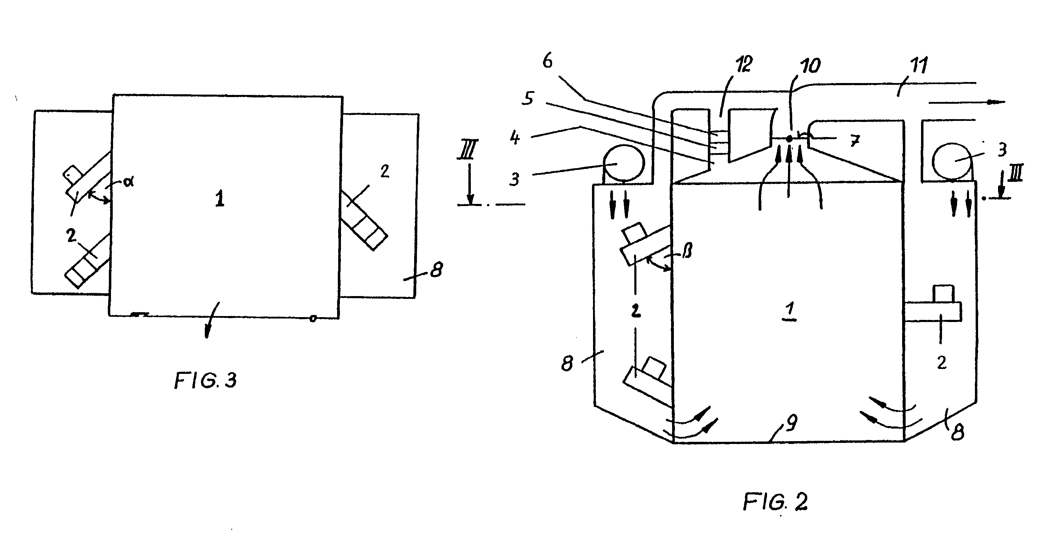

Aus fig. 3 ist ersichtlich, daß in Draufsicht die Hohlleiter 2 unter einem Winkel $\alpha$ von etwa 45° an der Seitenwand und in Vorderansicht unter einem Winkel $\beta$ von etwa 65° angeordnet sind. Es sind die Vektoren der elektrischen feldstärke $\varphi$ der, insbesondere über Hohlleiter 2 in den Hohlresonator 1 eingekoppelten Wellen orthogonal bzw. annähernd orthogonal zueinander angeordnet, wobei die Richtung eines jeden der drei E-Vektoren der eingekoppelten Wellen von der Richtung einer der Achsen des vom Resonator (Hohlresonator) 1 gebildeten räumlichen Koordinatensystems abweicht, dessen Koordinatenachsenrichtungen durch die drei senkrecht aufeinander stehenden Kanten des den Resonator 1 umschließenden rechteckigen Prismas gegeben sind. Die drei Magnetrons sichern eine über den Innenraum des Hohlresonators 1 gleichmäßig verteilte Leistungsdichte und das Desinfektionsgut (Sterilisationsgut) gelangt solcherart zu einer gleichmäßigen Erwärmung. Die drei auf den Hohlleitern angebrachten Magnetrons werden durch zwei Ventilatoren 3 gekühlt. Die Ventilatoren 3 erfüllen darüber hinaus die Aufgabe, in der in Fig. 2 dargestellten Weise den Innenraum des Hohlresonators 1 so mit Luft zu durchströmen, dorf der im Innenraum entstehende Wasserdampf zum geeigneten Zeitpunkt wieder ausgeblasen werden kann. Die Ventilatoren 3 fördern in Räume 8, in welchen sich die Magnetrons 2 befinden. Die Verbindung der Räume 8 mit dem Innenraum des Resonators 1 erfolgt über im Bereich des Bodens 9 des Resonators 1 befindliche Öffnungen. Der Innenraum des Resonators 1 ist über eine in einem Auslaßkanal 10 des Resonators 1 befindliche Stauklappe 7 mit einem Abluftkanal verbindbar, der in das Kanalnetz einmündet. Ist die Stauklappe 7 offen, so kann die von den Lüftern 3 in den Resonator 1 geförderte Luft über den Auslaßkanal 10 dem Kanalnetz über den Abluftkanal 11 zugeführt werden.

Weiters ist ein mit dem Innenraum des Resonators 1 verbundener Meßkanal 12 vorgesehen. Dieser Meßkanal 12 ist über einen kleinen Saugventilator 4 mit Luft aus dem Innenraum des Resonators 1 versorgbar. Der Meßkanal 12 führt dabei die Luft aus dem Innenraum des Resonators 1 über eine Heizeinrichtung 5 einem Feuchtesensor 6 zu. Die Heizeinrichtung 5 und der Feuchtesensor 6 sind in dem dargestellten Ausführungsbeispiel (Fig. 2) in dem Meßkanal 12 angeordnet. Die Luft in dem Meßkanal 12 wird dabei durch die Heizeinrichtung 5 auf eine konstante Temperatur vorgewärmt. Der Meßkanal 12 mündet in den Abluftkanal 11 ein, so daß auch die für Meßzwecke dem Innenraum 1 des Resonators entzogene Luft in den Abluftkanal 11 gelangt.

Der Feuchtesensor 6 kann in nicht dargestellter Weise mit einer Abschalteinrichtung für die Energieversorgung der Magnetrons 2 gekoppelt sein.

Im Innenraum des Resonators 1 befindet sich eine von einem Außenanschluß unabhängige Flüssigkeitsquelle, beispielsweise ein Beutel, der mit Wasser, gegebenenfalls zusätzlich mit Desinfektionsmittel und/oder Duftstoffen befüllt ist

Das zu desinfizierende bzw. sterilisierende Gut wird in einem Behältnis, das durch die Einwirkung von Mikrowellen in seiner Funktion nicht beeinträchtigt werden kann, und welches nach außen nicht dampfdicht verschlossen ist, in den Innenraum 1 des Resonators bei geöffneter Tür eingebracht. Zu Beginn des Desinfektionsvorganges werden die Lüfter 3 eingeschaltet und die Stauklappe 7 ist geöffnet. Über den kleinen Ventilator 4 wird aus dem Innenraum des Resonators 1 Luft angesaugt, durch die Heizeinrichtung 5 auf die gewählte konstante Temperatur gebracht und bei dieser Temperatur durch einen Sensor 6 der Feuchtigkeitsgehalt bestimmt. Die Stauklappe 7 wird geschlossen und dann werden die Magnetrons 2 eingeschaltet. Über den kleinen Ventilator 4 wird ständig eine bestimmte Luftmenge aus dem Innenraum des Resonators 1 entzogen und deren Feuchtegehalt bei konstanter Temperatur gemessen. Sobald der Feuchtegehalt auf einen sich vom Anfangsfeuchtegehalt vor der Einschaltung der Magnetrons 2 signifikant unterscheidenden Wert erhöht, läßt sich aus dieser Erhöhung schließten, daß das zu desinfizierende Gut mindestens die Kochtemperatur von Wasser erreicht hat. Dies erlaubt eine Aussage darüber, ob sich überhaupt desinfizierbares Gut im Innenraum des Resonators 1 befindet. Die Mikrowellenenergie wird nun eine festgesetzte Zeit einwirken gelassen, so daß eine Abtötung der wesentlichen Keim im Sinne einer Desinfektion sichergestellt wird. In der letzten Phase der Desinfektion wird die Stauklappe 7 wieder geöffnet, so daß die im Innenraum 1 entstandene Feuchtigkeit ausgeblasen werden kann. Die Magnetrons 2 werden abgeschaltet, wobei die beiden Ventilatoren 3 noch einige Zeit in Betrieb stehen, um die Restfeuchte aus dem Innenraum des Resonators 1 auszublasen. Dann kann die Türe des Resonators 1 geöffnet und das desinfizierte Gut mitsamt dem Behälter, in welchem es sich befindet, aus dem Resonator 1 entnommen werden. Soferne während der Einwirkung der Mikrowellen keine signifikante Erhöhung der Feuchtigkeit beobachtet werden kann, wird nach einer vorgegebenen Zeit die Abschaltung der Magnetrons 2 erfolgen.

**Patentansprüche**

1. Vorrichtung zum Erwärmen von Gegenständen durch Einwirken von mittels mehrerer Magnetrons erzeugten Mikrowellen, die über Wellenleiter in einen Raum eingespeist werden, in den die zu erwärmenden Gegenstände einbringbar sind, wobei die Wellenleiter an einen Hohlresonator (1) angeschlossen sind und der von einem rechteckigen Prisma gebildete Raum zur Aufnahme der zu erwärmenden Gegenstände vom Hohlresonator (1) umschlossen ist, wobei eines der Magnetrons (2) jeweils in den von den Wellen des bzw. der anderen Magnetrons (2) freien Raumbereich strahlt und die Vektoren der elektrischen Feldstärke ($\varphi$) der in den Hohlresonator (1) eingekoppelten Wellen orthogonal bzw. annähernd orthogonal zueinander angeordnet sind und die Richtung eines jeden der Vektoren ($\varphi$) der eingekoppelten Wellen von der Richtung einer der Achsen des durch drei senkrecht aufeinanderstehenden Kanten des den Raum zur Aufnahme der zu erwärmenden Gegenstände bildenden rechteckigen Prismas bestimmten räumlichen Koordinatensystems abweicht, so daß im Hohlresonator (1) kalte Stellen vermieden werden und das in den Hohlresonator (1) eingebrachte Gut zur Gänze der Einwirkung der Mikrowellen ausgesetzt wird.

2. Vorrichtung nach Anspruch 1, dadurch gekennzeichnet, daß drei Magnetrons (2) vorgesehen sind, von welchen zwei an einer Seitenwand des Hohlresonators (1) liegen und das dritte Magnetron (2) an der dieser Wand gegenüberliegenden Wand des Hohlresonators liegt.

3. Vorrichtung nach Anspruch 1 oder 2, dadurch gekennzeichnet, daß der Innenraum des Hohlresonators (1) über, insbesondere im Bereich des Bodens (9) des Hohlresonators (1I) befindliche, Öffnungen mit Außenräumen (8) verbunden ist, in welchen insbesondere Raumluft ansaugende Ventilatoren (3) zur Belüftung der Außenräume (8) und der dort angeordneten Magnetrons (2) samt zugehörigen Hohlleitern, über welche die Wellen in den Hohlresonator (1) eingekoppelt werden, angeordnet sind.

4. Vorrichtung nach einem der Ansprüche 1 bis 3, dadurch gekennzeichnet, daß der Innenraum des Hohlresonators (1) über einen, bevorzugt in das Kanalnetz einmündenden, Auslaßkanal (10) mit einem Abluftkanal (11) verbindbar ist, wobei im Auslaßkanal (10) eine Stauklappe (7) angeordnet ist.

5. Vorrichtung nach einem der Ansprüche 1 bis 4, dadurch gekennzeichnet, daß mit dem Innenraum des Hohlresonators (1) ein Meßkanal (12) verbunden ist, der aus dem Innenraum des Hohlresonators (1), insbesondere über einen Saugventilator (4), mit Luft versorgbar ist und die Luft über eine Heizeinrichtung (5) einem Feuchtesensor (6) zuführt.

6. Vorrichtung nach Anspruch 5, dadurch gekennzeichnet, daß die Heizeinrichtung (5) und der Feuchtesensor (6) in dem Meßkanal (12) angeordnet sind.

7. Vorrichtung nach Anspruch 6, dadurch gekennzeichnet, daß der Meßkanal (12) in den Abluftkanal (11) mündet.

8. Vorrichtung nach einem der Ansprüche 5 bis 7, dadurch gekennzeichnet, daß der Feuchtesensor (6) mit einer Abschalteinrichtung für die Energieversorgung der Magnetrons (2) gekoppelt ist.

9. Vorrichtung nach einem der Ansprüche 1 oder 2, dadurch gekennzeichnet, daß mit dem Innenraum des Hohlresonators (1) ein Thermorelais (13) zum Schutz gegen Überhitzung verbunden ist.

10. Vorrichtung nach Anspruch 1, 2 oder 9, bei der im Innenraum des Hohlresonators Dampf erzeugt wird, dadurch gekennzeichnet, daß ein Sicherheitsventil (15) zur Sicherung des Hohlresonators (1) gegen inneren Überdruck vorgesehen ist.

11. Vorrichtung nach einem der Ansprüche 1 bis 10, dadurch gekennzeichnet, daß im Innenraum des Hohlresonators (1) eine autarke, somit von einem Außenanschluß unabhängige Flüssigkeitsquelle, z.B. in Form eines mit Wasser, gegebenenfalls zusätzlich mit Desinfektionsmittel und/oder Duftstoffen befüllten Beutels, sich befindet.

## Claims

1. Apparatus for heating articles by the action of microwaves produced by a plurality of magnetrons which are fed via wave guides into a space into which the articles to be heated may be introduced, whereby the wave guides are connected to a resonant cavity (1) and the space for receiving the articles to be heated, which is defined by a rectangular prism, is surrounded by the resonant cavity (1), whereby one of the magnetrons (2) radiates into the space region free of the waves from the respective other magnetron(s) (2) and the vectors of the electric field strength ($\varphi$) of the waves coupled in in the resonant cavity (1) are disposed orthogonally or approximately orthogonally to one another and the direction of one of each of the vectors ($\varphi$) of the coupled-in waves deviates from the direction of one of the axes of the spatial coordinate system defined by three perpendicularly extending edges of the rectangular prism defining the space for receiving the articles to be heated so that cold spots are prevented in the resonant cavity (1) and the material introduced into the resonant cavity (1) is subjected in its entirety to the action of the microwaves.

2. Apparatus as claimed in Claim 1, characterised in that three magnetrons (2) are provided, of which two are adjacent to one side wall of the resonant cavity (1) and the third magnetron (2) is adjacent to the wall of the resonant cavity opposite to this wall.

3. Apparatus as claimed in Claim 1 or 2, characterised in that the interior of the resonant cavity (1) is connected via openings, particularly situated in the vicinity of the floor (9) of the resonant cavity (1), to external spaces (8) in which fans (3), particularly which suck in room air, are disposed for ventilating the external spaces (8) and the magnetron (2) arranged there together with associated tubular guides by which the waves are coupled into the resonant cavity (1).

4. Apparatus as claimed in one of Claims 1 to 3, characterised in that the interior of the resonant cavity (1) may be connected to an exhaust passage (11) via an outlet passage which preferably discharges into the sewage network, a throttling flap (7) being disposed in the outlet passage (10).

5. Apparatus as claimed in one of Claims 1 to 4, characterised in that connected to the interior of the resonant cavity (1) there is a measuring passage (12) which may be supplied with air from the interior of the resonant cavity (1), particularly via a suction fan (4), and supplies the air via a heating device (5) to a moisture sensor (6).

6. Apparatus as claimed in Claim 5, characterised in that the heating device (5) and the moisture sensor (6) are disposed in the measuring passage (12).

7. Apparatus as claimed in Claim 6, characterised in that the measuring passage (12) discharges into the exhaust passage (11).

8. Apparatus as claimed in one of Claims 5 to 7, characterised in that the moisture sensor (6) is coupled to a cut-out device for the energy supply to the magnetron (2).

9. Apparatus as claimed in one of Claims 1 or 2, characterised in that a thermal relay (13) is connected to the interior of the resonant cavity (1) for protecting against overheating.

10. Apparatus as claimed in Claim 1, 2 or 9 in which steam is produced in the interior of the resonant cavity, characterised in that a safety valve (15) is provided for protecting the resonant cavity (1) against an internal overpressure.

11. Apparatus as claimed in one of Claims 1 to 10, characterised in that situated in the interior of the resonant cavity (1) there is a liquid source which is autonomous and thus independent of an external connection, e.g. in the form of a bag filled with water and optionally additionally with disinfectant and/or fragrances.

## Revendications

1. Dispositif pour chauffer des objets par l'action de micro-ondes produites par plusieurs magnétrons et introduites par des guides d'ondes dans un espace dans lequel on peut placer les objets à chauffer, les guides d'ondes étant branchés sur un résonnateur à cavité (1) et l'espace formé par un prisme rectangulaire pour recevoir les objets à chauffer étant entouré par le résonnateur à cavité (1), un des magnétrons (2) émettant respectivement dans une zone non atteinte par les ondes du ou des autres magnétrons (2), les vecteurs de l'intensité de champ électrique (φ) des ondes introduites dans le résonnateur à cavité (1) présentant une disposition orthogonale ou approximativement orthogonale les uns par rapport aux autres, et la direction de chacun des vecteurs (φ) des ondes introduites divergeant de la direction de l'un des axes du système de coordonnées spatiales défini par trois arêtes disposées l'une par rapport à l'autre dans le sens perpendiculaire appartenant au prisme rectangulaire qui constitue l'espace recevant les objets à chauffer de telle sorte que l'on puisse éviter d'avoir des portions froides à l'intérieur du résonnateur à cavité (1), et que l'objet placé dans le résonnateur (1) soit exposé à la totalité de l'action des micro-ondes.

2. Dispositif selon la revendication 1 caractérisé en ce que trois magnétrons (2) sont prévus, dont deux sont placés sur une paroi latérale du résonnateur à cavité (1) et le troisième magnétron (2) est placé sur la paroi latérale opposée du résonnateur à cavité.

3. Dispositif selon la revendication 1 ou 2 caractérisé en ce que l'espace intérieur du résonnateur à cavité (1) est relié, par l'intermédiaire d'ouvertures situées en particulier dans la zone du fond (9) du résonnateur à cavité (1), à des espaces extérieurs (8), dans lesquels sont disposés en particulier des ventilateurs (3) aspirant l'air de l'espace pour la ventilation des espaces extérieurs (8) et des magnétrons (2) qui y sont placés, y compris les guides creux associés, par l'intermédiaire desquels les ondes sont introduites dans le résonnateur à cavité (1).

4. Dispositif selon l'une quelconque des revendications 1 à 3, caractérisé en ce que l'espace intérieur du résonnateur à cavité (1) peut être relié à un canal d'évacuation d'air (11) par l'intermédiaire d'un canal de sortie (10) débouchant de préférence dans le système de canaux, une vanne de retenue (7) étant disposée dans le canal de sortie (10).

5. Dispositif selon l'une quelconque des revendications 1 à 4 caractérisé en ce qu'un canal de mesure (12) est relié à l'espace intérieur du résonnateur à cavité (1), pouvant être alimenté en air à partir de l'espace intérieur du résonnateur (1) en particulier par l'intermédiaire d'un ventilateur aspirant (4), et qui amène l'air vers un capteur d'humidité (6) par l'intermédiaire d'un dispositif de chauffage (5).

6. Dispositif selon la revendication 5 caractérisé en ce que le dispositif de chauffage (5) et le capteur d'humidité (6) sont placés dans le canal de mesure (12).

7. Dispositif selon la revendication 6 caractérisé en ce que le canal de mesure (12) débouche dans le canal d'évacuation d'air (11).

8. Dispositif selon l'une quelconque des revendications 5 à 7 caractérisé en ce que le capteur d'humidité (6) est accouplé à un dispositif de coupure pour l'alimentation en énergie des magnétrons (2).

9. Dispositif selon l'une quelconque des revendications 1 ou 2 caractérisé en ce qu'un relais thermique (13) est relié à l'espace intérieur du résonnateur à cavité (1) pour le protéger d'une surchauffe.

10. Dispositif selon la revendication 1, 2 ou 9 dans lequel de la vapeur est produite dans l'espace intérieur du résonnateur à cavité, caractérisé en ce qu'une soupape de sécurité (15) est prévue pour protéger le résonnateur (1) contre une surpression intérieure.

11. Dispositif selon l'une quelconque des revendications 1 à 10, caractérisé en ce qu'est disposée, dans l'espace intérieur du résonnateur à cavité (1), une source de liquide autonome et par conséquent indépendante d'un raccord vers l'extérieur, se présentant par exemple sous la forme d'un sac rempli d'eau et éventuellement également d'agent désinfectant et/ou d'une substance odorante.

FIG.1

FIG. 3

FIG. 2

EP 0 287 549 B1